# FASCICULE DE BREVET EUROPEEN

(11) **EP 4 153 049 B1**
(45) Date de publication et mention de la délivrance du brevet: **28.02.2024**
(21) Numéro de dépôt: 22729667.0
(22) Date de dépôt: 19.05.2022
(51) Int. Cl.: A61B 5/16, A61B 5/00

(54) **UN SYSTÈME ET UN PROCÉDÉ DE MESURE DE RÉACTIONS D'UN SUJET**
SYSTEM UND VERFAHREN ZUR MESSUNG VON REAKTIONEN EINER PERSON
SYSTEM AND METHOD FOR MEASURING REACTIONS OF A SUBJECT

(30) Priorité: 19.05.2021 BE 202105404
(43) Date de publication de la demande: 29.03.2023
(73) Titulaire: P³Lab, 1341 Ceroux-Mousty (BE)
(72) Inventeur: DAYE, Pierre Martin Jack Gérard, 1420 Braine-l'alleud (BE)
(74) Mandataire: Gevers Patents
(86) Numéro de dépôt international: PCT/EP2022/063613
(87) Numéro de publication internationale: WO 2022/243454

(56) Documents cités:
- EP-A1- 2 358 261
- EP-A1- 2 358 261
- EP-A1- 3 064 130
- PORTENGEN BRENDAN L ET AL: "Blind spot and visual field anisotropy detection with flicker pupil perimetry across brightness and task variations", VISION RESEARCH, ELSEVIER, AMSTERDAM, NL, vol. 178, 5 novembre 2020 (2020-11-05), pages 79-85, XP086433965, ISSN: 0042-6989, DOI: 10.1016/J.VISRES.2020.10.005 [extrait le 2020-11-05] cité dans la demande

## Description

### Domaine technique

La présente invention concerne un système et un procédé de mesure de réactions d'un sujet.

### Art antérieur

Pour certaines applications, par exemple médicales, on désire suivre le mouvement de l'œil d'un patient (« eyetracking » en anglais) ; en fonction du mouvement de l'œil réagissant typiquement à un stimulus visuel, on peut par exemple déduire certaines informations neurologiques du patient.

En général, un écran est utilisé pour engendrer le stimulus visuel et « forcer » l'œil à suivre, regarder un motif, un ou plusieurs pixels. Une ou plusieurs caméras capturent des images de l'œil à différents moments. A titre d'exemple, le document EP2358261 décrit un système pour présenter des stimuli aux yeux d'un sujet et enregistrer les réponses des pupilles. La séquence de stimuli peut se présenter sous la forme d'un signal vidéo qui est affiché sur des écrans LCD respectifs, à une fréquence de 60 images par seconde. Des détecteurs échantillonnent les réponses des pupilles de chacun des yeux du sujet à une fréquence de 30 images par seconde. L'une des fréquences des écrans et des caméras est le double de l'autre, mais il n'est pas indiqué dans ce document comment synchroniser l'affichage sur les écrans LCD et la capture par les détecteurs. Ainsi cette technique présente un désavantage : il existe une incertitude temporelle entre un affichage d'un stimuli par les écrans et la prise d'images de l'œil par les détecteurs. Cette incertitude se répercute naturellement sur la mesure des réactions du sujet - oculaires en particulier - et le traitement des résultats. Ceci est important dans le traitement des résultats de tests car cela impacte la complexité des calculs à mettre en oeuvre pour traiter les résultats - et donc la puissance et le coût des calculateurs.

Le document Portengeen Brendan L et AI : « Blind spot and visual field anistotropy détection with flicker pupil perimetry across brightness and task validations », Vision Research, Elsevier, Amsterdam, NL, p. 80-81 (XP086433965) décrit l'utilisation du périmètre de pupille comme moyen de mesure de la sensibilité à travers le champ visuel. Il est prévu une installation comprenant un ordinateur sur lequel des stimuli sont affichés et une caméra enregistrant la taille de la pupille et l'angle du regard.

Le document EP3064130 décrit un dispositif de mesure de réactions d'un sujet. Le dispositif comprend un système de détection avec des capteurs du regard, un système de simulation avec un écran positionné devant les yeux de sujet pour lui présenter des informations visuelles et un système de contrôle avec une unité d'acquisition et une horloge. L'unité d'acquisition est configurée pour recevoir des signaux du système de stimulation et des signaux du système de détection et pour, ensuite, horodater ces signaux avec un signal d'horloge de l'horloge. L'horodatage a donc lieu en aval de l'acquisition des signaux. Le document US20150297075 décrit une collecte de données liées à l'affichage de stimuli et des données liées aux yeux d'un patient. Ce document décrit l'association de ces données acquises à des informations liées au temps. De même que pour le document EP3064130, L'horodatage dans le document US20150297075 a lieu en aval de l'acquisition des signaux. Ainsi, non seulement ces documents ne décrivent pas de fréquences synchronisées mais encore, ces documents présentent le désavantage d'avoir une incertitude temporelle entre l'affichage des informations visuelles et la détection par les capteurs. Comme précédemment, cette incertitude se répercute naturellement sur la mesure des réactions oculaire du sujet et le traitement des résultats.

Il y a un besoin pour un système de mesure de réaction d'un sujet qui permette d'améliorer la précision de la mesure des réactions du sujet à des stimuli.

### Exposé de l'invention

Un objet de la présente invention est d'améliorer la précision de la mesure des réactions du sujet aux stimuli.

À cet effet, l'invention propose un système de mesure de réactions d'un sujet selon la revendication 1.

Grâce au système de la présente invention, quand on prend une image avec le système de l'invention, on peut savoir avec certitude quel(s) pixel(s) de l'écran ont été rafraîchis. Il est possible de déterminer à quel(s) pixel(s) rafraîchi(s) de l'écran affichant les stimuli correspond une certaine image capturée par le capteur. Il y a donc une certitude temporelle dans la correspondance des données fournies par l'écran et le capteur car la même unité logique et l'horloge dont elle est pourvue commandent et synchronisent la valeur des fréquences et la phase du rafraîchissement de la capture. En particulier, grâce à l'invention, le post-traitement pour accorder les images du sujet avec l'apparition des stimuli est limité, ce qui améliore la précision de la mesure de réaction du sujet aux stimuli.

Selon les modes particuliers de réalisation, l'invention peut comprendre une ou plusieurs caractéristiques suivantes selon toute combinaison techniquement possibles :
- Le multiple entier est de 1 et l'unité logique est configurée pour commander le rafraîchissement des pixels de l'écran et la capture des images du sujet par le capteur à la même fréquence.
- La première fréquence est plus rapide que la deuxième fréquence, la première fréquence étant un multiple entier de la deuxième fréquence.
- La deuxième fréquence est plus rapide que la première fréquence, la deuxième fréquence étant un multiple entier de la première fréquence.
- L'unité logique est raccordée directement à l'écran et au capteur.
- Les images capturées par le capteur sont des images d'au moins un oeil du sujet.
- Le système de mesure comprend deux capteurs sous forme de caméras, chaque capteur capturant des images d'un oeil respectif du sujet.
- Le au moins un capteur et l'écran sont à balayage de gauche à droite et de haut en bas.

L'invention a également pour objet un procédé de mesure de réactions d'un sujet, comprenant les étapes suivantes :
- la fourniture d'un écran, d'au moins un capteur, et d'une unité logique comprenant une horloge centrale,
- l'affichage par l'écran d'une pluralité de stimuli par un rafraîchissement de pixels de l'écran, les stimuli générant des réactions du sujet, et la capture par le capteur d'images du sujet en réaction aux stimuli,
le rafraîchissement des pixels de l'écran et la capture des images du sujet par le capteur étant commandés par l'unité logique respectivement à une première fréquence et une deuxième fréquence, l'une des fréquences étant un multiple entier de l'autre fréquence. En outre, l'horloge centrale est configurée pour commander le rafraîchissement de pixels de l'écran et la capture des images par le capteur, dans lequel
- le commandement des fréquences du rafraîchissement des pixels de l'écran et de la capture des images par le capteur est effectué uniquement par l'unité logique,
- le commandement du rafraîchissement des pixels de l'écran et de la capture des images par le ou les capteurs est effectuée uniquement par l'horloge centrale de l'unité logique, de sorte qu'il y a une relation bijective entre l'instant où un pixel du capteur est acquis et un pixel de l'écran est rafraîchi.

Selon les modes particuliers de réalisation :
- le multiple entier est de 1, l'unité logique commandant le rafraîchissement des pixels de l'écran et la capture des images du sujet par le capteur à la même fréquence.
- la première fréquence est plus rapide que la deuxième fréquence, la première fréquence étant un multiple entier de la deuxième fréquence.
- la deuxième fréquence est plus rapide que la première fréquence, la deuxième fréquence étant un multiple entier de la première fréquence.
- l'étape de fourniture comprend la fourniture de deux capteurs sous forme de caméras.
- l'étape de capture comprend la capture par chaque capteur d'images d'un oeil respectif du sujet.

Les modes de réalisation et les avantages du système de mesure selon l'invention se transposent *mutatis mutandis* au procédé de mesure, au programme d'ordinateur et au support informatique lisible par un ordinateur selon l'invention.

### Brève description des figures

D'autres caractéristiques et avantages de la présente invention apparaîtront à la lecture de la description détaillée qui suit pour la compréhension de laquelle on se reportera à la figure annexée :
- la figure 1 illustre une vue schématique un système de mesure selon l'invention ;
- la figure 2 illustre une vue schématique du fonctionnement du système selon l'invention selon un exemple ;
- la figure 3 illustre une vue schématique du fonctionnement du système selon l'invention selon un autre exemple.

Le dessin de la figure n'est pas à l'échelle. Des éléments semblables sont en général dénotés par des références semblables sur la figure. Dans le cadre du présent document, les éléments identiques ou analogues peuvent porter les mêmes références. En outre, la présence de numéros ou lettres de référence à la figure ne peut être considérée comme limitative, y compris lorsque ces numéros ou lettres sont indiqués dans les revendications.

### Description détaillée de modes de réalisation de l'invention

Cette partie présente une description détaillée de modes de réalisation préférés de la présente invention. Cette dernière est décrite avec des réalisations particulières et des références à une figure mais l'invention n'est pas limitée par celle-ci. En particulier, la figure décrite ci-dessous n'est que schématique et n'est pas limitative.

L'usage, dans ce document, du verbe « comprendre », de ses variantes, ainsi que ses conjugaisons, ne peut en aucune façon exclure la présence d'éléments autres que ceux mentionnés. L'usage, dans ce document, de l'article indéfini « un », « une », ou de l'article défini « le », « la » ou « l' », pour introduire un élément n'exclut pas la présence d'une pluralité de ces éléments.

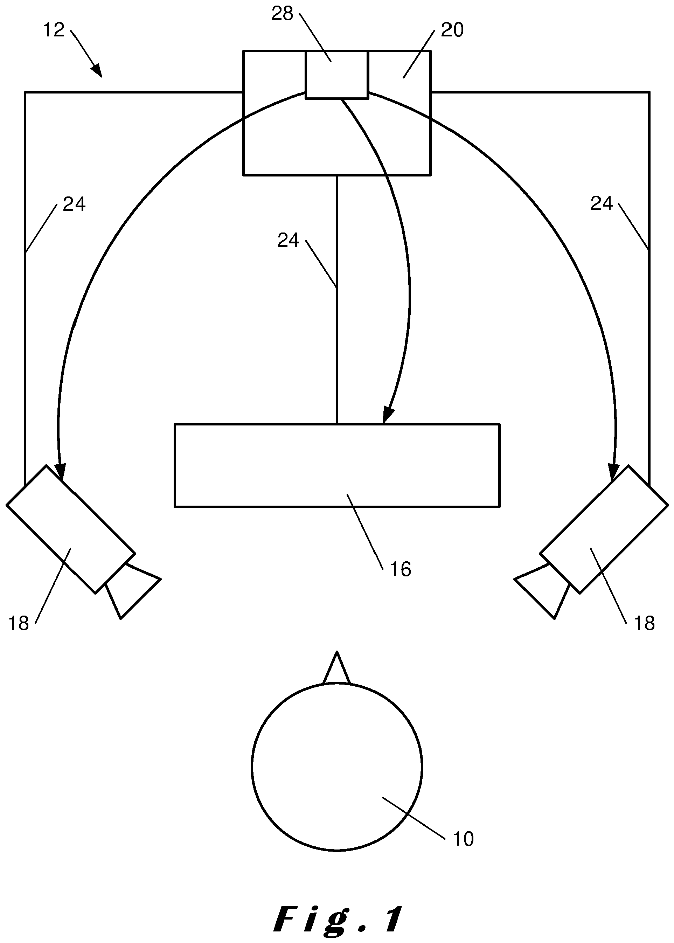

La figure 1 illustre un système de mesure 12 de réaction d'un sujet 10. Le système de mesure 12 comprend un écran 16 apte à afficher une pluralité de stimuli par un rafraîchissement de pixels de l'écran 16. Les stimuli affichés par l'écran 16 sont aptes à générer des réactions du sujet 10. Les réactions du sujet 10 sont des réactions physiques, notamment de son visage et en particulier les réactions d'au moins un oeil du sujet 10. Les réactions du sujet 10 peuvent comprendre par exemple le mouvement de sa pupille, ou l'augmentation ou la diminution de la taille de sa pupille. Le système de mesure 12 comprend aussi au moins un capteur 18 apte à capturer des images du sujet 10 en réaction aux stimuli. Le système de mesure 12 comprend également une unité logique 20 configurée pour commander le rafraîchissement des pixels de l'écran 16 à une première fréquence et la capture des images du sujet 10 par le capteur 18 à une deuxième fréquence. L'une des fréquences est un multiple entier de l'autre fréquence. La capture des images du sujet 10 par le ou les capteurs 18 étant à une fréquence qui est un multiple entier de la fréquence de rafraichissement des pixels de l'écran 16 (ou inversement), le système permet d'obtenir un nombre entier d'images capturées par le ou les capteurs 18.

L'unité logique 20 comprend en outre une horloge centrale 28 configurée pour commander le rafraîchissement des pixels de l'écran 16 et la capture des images par le capteur 18. En d'autres termes, l'horloge centrale 28 est configurée pour commander le déclenchement du rafraîchissement des pixels de l'écran 16 et de la capture des images par le capteur 18. En d'autres termes encore, l'horloge centrale 28 est configurée pour déclencher le rafraîchissement des pixels de l'écran 16 et la capture des images par le capteur 18. Le fait d'utiliser la même horloge (une horloge unique), le rafraîchissement des pixels de l'écran 16 et la capture des images par les capteurs 18 sont déclenchés (ou activés) en même temps. Les flancs montants de l'horloge sont utilisés pour s'assurer que la présentation du premier pixel de l'écran commence en même temps que la capture par le capteur (et donc par exemple, en même temps que la capture du premier pixel de la première image du capteur sous forme de caméra). De plus, de par la commande (c'est-à-dire le déclenchement) par l'horloge 28, le rafraîchissement des pixels de l'écran 16 et la capture des images par les capteurs 18 sont des informations qui sont horodatées dès leur commande (en amont de leur commande). Une information temporelle leur est attribuée dès leur commande. On obtient donc une information temporelle associée au rafraîchissement des pixels de l'écran 16 et à la capture des images par le ou les capteurs 18 qui est déclenchée en même temps pour le rafraîchissement et la capture. Le déclenchement du rafraîchissement des pixels de l'écran 16 et de la capture des images par le capteur 18 est sous la commande exclusive de l'horloge centrale 28, comme illustré par les flèches sur la figure 1. Le capteur 18 est en mesure de capturer des images qu'une fois l'horloge centrale 28 qui commande le déclenchement du rafraîchissement des pixels de l'écran 16 est activée. Par ailleurs, la fréquence de l'horloge 28 correspond à un multiple entier de la fréquence de l'écran et du ou des capteurs. Ainsi, on garantit le fait que la présentation du premier pixel de la deuxième image du stimuli commence exactement à un flanc montant de l'horloge et que la relation temporelle entre l'acquisition des pixels par les capteurs et la présentation des stimuli visuels par l'écran est conservée.

Ainsi, d'une part l'unité logique 20 commande les première et deuxième fréquences applicables au rafraîchissement des pixels de l'écran 16 et à la capture des images du sujet 10 par le ou les capteurs 18 et d'autre part, l'unité logique 20 comprend l'horloge centrale 28 déclenchant le rafraîchissement de pixels de l'écran 16 et la capture des images. L'unité logique 20 et l'horloge 28 synchronisent donc la valeur des fréquences appliquées et la phase du déclenchement du rafraîchissement des pixels de l'écran 16 et de la capture des images par le ou les capteurs 18. On obtient une relation bijective entre l'instant où une image est capturée par le ou les capteurs et un pixel de l'écran est affiché, rafraîchi. Ceci permet alors de mieux coordonner les mesures des réactions du sujet par rapport à l'affichage des stimuli et donc de réduire la puissance de calcul pour le traitement des résultats. Ceci améliore la précision de la mesure des réactions du sujet et simplifie donc les calculs. Le système 12 permet ainsi d'éviter toute incertitude temporelle entre l'affichage d'un stimuli sur l'écran et la prise d'images de l'œil par la ou les capteurs. En d'autres termes, le système permet de lier une image du sujet - l'œil par exemple - avec un affichage donné de stimuli sur l'écran. Le rafraîchissement et la capture à des fréquences respectives qui sont un multiple entier l'une de l'autre et la commande (ou la commande du déclenchement) du rafraichissement et de la capture par une même horloge permettent d'éviter une désynchronisation entre le fonctionnement de l'écran et du ou des capteurs, ce qui permet de réduire alors la puissance de calcul pour traiter les informations. On augmente ainsi la qualité des mesures de réactions du sujet.

Cette architecture permet d'avoir un alignement temporel entre les enregistrements et la présentation des stimuli à l'écran, car le déclenchement des actions est concomitant et les fréquences sont multiples l'une de l'autre. Cela permet une synchronisation précise facilitant l'analyse des résultats. L'utilisateur n'aura pas à s'appuyer sur des sondes externes ou des astuces logicielles pour aligner les stimuli et les enregistrements. Par conséquent, même si l'écran par exemple a une fréquence plus basse, le système donne des mesures précises des temps de mouvement des yeux (par exemple, la latence par rapport à un changement de position cible). L'affichage et l'acquisition sont coordonnées dans le temps.

Selon un mode de réalisation, le multiple entier peut être de 1 ; l'unité logique 20 est configurée pour commander le rafraîchissement des pixels de l'écran 16 et la capture des images du sujet 10 par le capteur 18 à la même fréquence. La première fréquence et la deuxième fréquence sont les mêmes. La commande de rafraîchissement et la commande de la capture des images sont alors simultanées. Ceci permet de réduire encore la puissance de calcul pour traiter les informations.

Selon un autre mode de réalisation, l'une ou l'autre des première et deuxième fréquences est plus rapide que l'autre, la fréquence la plus rapide étant un multiple entier de l'autre fréquence. Cela permet d'avoir un écran ou un capteur qui soient moins performants et donc moins onéreux tout en permettant un traitement aisé des informations. Par exemple, la première fréquence de rafraîchissement des pixels de l'écran est plus rapide que la deuxième fréquence de capture des images par le capteur, la première fréquence étant un multiple entier de la deuxième fréquence. Alternativement, la deuxième fréquence de capture des images par le capteur est plus rapide que la première fréquence de rafraîchissement des pixels de l'écran, la deuxième fréquence étant un multiple entier de la première fréquence. Ceci permet de multiplier les images de réactions du sujet afin d'obtenir des mesures plus précises.

Le multiple entier n entre les deux fréquences est choisi dans groupe 11, 2, 3, 4, 5, 6, 7, 8, 9, 10, 11, 12,...}. Pour une valeur de 1, les deux fréquences sont les mêmes. A titre d'exemple, les pixels de l'écran 16 sont rafraîchis à une première fréquence comprise entre 50Hz et 500 Hz. A titre d'exemple, dans l'hypothèse de la deuxième fréquence de capture d'images plus rapide que la première fréquence de rafraîchissement des pixels de l'écran, la première fréquence peut être de 50 ou 60 Hz, la deuxième fréquence respectivement de 500 ou 720 Hz et le multiple entier n de 10 ou 12 respectivement; dans l'hypothèse de la première fréquence de rafraîchissement des pixels de l'écran plus rapide que la deuxième fréquence de capture d'images, la première fréquence peut être de 360 Hz, la deuxième fréquence de 40 Hz et le multiple entier n de 9.

L'écran 16 a un sens large dans la présente demande. Selon un mode de réalisation, l'écran 16 est un dispositif d'affichage apte à émettre de la lumière lui-même, par exemple un écran LED (abréviation de « diode électroluminescente »). Selon un autre mode de réalisation, l'écran 16 est une surface apte à réfléchir de la lumière projetée sur la surface, par exemple la lumière projetée par un rétroprojecteur. De préférence, le rafraîchissement de l'écran 16 est à balayage. Ceci permet de maîtriser les informations de rafraîchissement des pixels et de faciliter le traitement des résultats. En particulier, le balayage est de gauche à droite et de haut en bas.

Le ou les capteur 18 sont typiquement une caméra. La caméra est opérationnelle dans les longueurs d'onde de la lumière visible et / ou de la lumière infrarouge et / ou de la lumière ultraviolette. La caméra est pourvue de pixels pour la capture des images. Les images sont capturées sous forme d'un flux vidéo par la caméra. Les images capturées par le capteur 18 sont en particulier des images d'au moins un oeil du sujet 10. Selon un mode de réalisation représenté sur la figure 1, le système de mesure 12 comprend plusieurs capteurs, de préférence deux capteurs 18 sous forme de caméras, capturant les images à la même deuxième fréquence. Chaque capteur 18 capture des images d'un oeil respectif du sujet 10. La présence de deux caméras permet de focaliser une caméra sur chaque oeil, augmentant ainsi la résolution spatiale. Selon un autre mode de réalisation, le système de mesure 12 comprend un seul capteur 18, par exemple également sous forme de caméra. Le seul capteur 18 est apte à capturer les images de deux yeux du sujet 10 à la deuxième fréquence. De préférence, la capture par le ou les capteurs 18 est à balayage. Ceci permet de maîtriser les informations de capture et de faciliter le traitement des résultats. En particulier, le balayage est de gauche à droite et de haut en bas. La capture par balayage, et de gauche à droite et de haut en bas, est en particulier applicable au/aux capteur(s) sous forme de caméra.

Le rafraîchissement des pixels de l'écran 16 et de la capture des images du sujet 10 par le ou les capteurs 18 à des fréquences qui sont un multiple entier l'une de l'autre permet d'éviter un désaccord entre l'affichage des stimuli sur l'écran qui se rafraîchirait à une première fréquence et la capture des images par le ou les capteurs à une deuxième fréquence qui ne soit pas un multiple entier de la première fréquence. Le système permet d'éviter le traitement complexe des informations.

L'unité logique 20 est configurée pour commander directement le rafraîchissement des pixels de l'écran 16 et la capture des images du sujet 10 par le ou les capteurs 18 à une fréquence respective, unique ou multiple entier l'une de l'autre. Le commandement des fréquences du rafraîchissement des pixels de l'écran 16 et de la capture des images par le capteur 18 est effectué uniquement par l'unité logique 20. En d'autres termes, l'écran 16 lui-même est dépourvu de composant apte à commander la fréquence de rafraîchissement des pixels de l'écran 16, et/ou le capteur 18 lui-même est dépourvu de composant apte à commander la fréquence de capture des images par le capteur 18. Également, le commandement (du déclenchement) du rafraîchissement des pixels de l'écran 16 et/ou de la capture des images par le ou les capteurs 18 est effectuée uniquement par l'horloge centrale 28 de l'unité logique 20, comme illustré par les flèches sur la figure 1. En d'autres termes, l'écran 16 et/ou le capteur 18 sont dépourvus d'horloge apte à effectuer le déclenchement du rafraîchissement de l'écran 16 et de la capture des images par le capteur 18.

Selon un mode de réalisation, l'unité logique 20 est raccordée directement à l'écran 16 et au capteur 18. Autrement dit, entre l'unité logique 20 et l'écran 16 le système de mesure 12 comprend seulement un câble 24. Le système de mesure 12 est dépourvu de composant apte à effectuer un traitement numérique ou une commande numérique entre l'unité logique 20 et l'écran 16. Entre l'unité logique 20 et le ou les capteurs 18, le système de mesure 12 comprend seulement un câble 24. Le système de mesure 12 est dépourvu de composant apte à effectuer un traitement numérique ou une commande numérique entre l'unité logique 20 et le capteur 18.

L'invention concerne aussi un procédé de mesure de réactions d'un sujet 10. En premier lieu, un utilisateur fournit un écran 16, au moins un capteur 18, et une unité logique 20 comprenant une horloge centrale 28 tels que décrits plus haut. Ensuite, l'unité logique 20 commande le rafraîchissement des pixels de l'écran 16 et la capture des images du sujet 10 par le ou les capteurs 18 à des fréquences respectives qui sont un multiple entier l'une de l'autre. Egalement, l'horloge centrale 28 est configurée pour commander le rafraîchissement de pixels de l'écran 16 et la capture des images par le ou les capteurs 18. L'horloge centrale 28 est configurée pour commander le déclenchement du rafraîchissement de pixels de l'écran 16 et la capture des images par le ou les capteurs 18. L'horloge centrale 28 est configurée pour déclencher le rafraîchissement de pixels de l'écran 16 et la capture des images par le ou les capteurs 18. Le rafraîchissement de pixels de l'écran 16 affiche une pluralité de stimuli sur l'écran 16, par exemple une série d'images. Les stimuli génèrent des réactions du sujet 10, en particulier des réactions oculaires du sujet 10, par exemple le mouvement de la pupille du sujet 10. Le capteur 18 capture des images du sujet 10 en réaction aux stimuli. Le rafraîchissement et la capture sont à des fréquences respectives qui sont un multiple entier l'une de l'autre et leur déclenchement est commandé par l'horloge 28. Le rafraîchissement et la capture ont une valeur de fréquences et une phase de déclenchement qui sont synchronisées. Ceci permet de réduire alors la puissance de calcul pour traiter les informations et d'augmenter la qualité des mesures de réactions du sujet. L'unité logique 20 est configurée pour commander le rafraîchissement des pixels de l'écran 16 à une première fréquence définie tandis que la capture des images du sujet 10 par le ou les capteurs 18 se fait à une deuxième fréquence, l'une des fréquences étant un multiple entier de l'autre fréquence.

Selon un mode de réalisation, les pixels de l'écran 16 sont rafraîchis et les images du sujet 10 sont capturées par le capteur 18 simultanément à une fréquence unique. Ainsi, les images capturées par le ou les capteurs 18 représentent la réaction synchronisée de l'œil aux stimuli présentés sur l'écran 16. Le procédé permet d'éviter le traitement complexe des informations. Selon le procédé, l'une ou l'autre de la première ou de la deuxième fréquences peut être la plus rapide, la fréquence la plus rapide étant un multiple entier de la fréquence la plus lente.

Lorsque le système de mesure 12 comprend plusieurs capteurs, par exemple deux capteurs 18, le procédé de mesure comprend la capture par chaque capteur 18 d'images d'un oeil respectif du sujet 10 selon une deuxième fréquence identique entre la pluralité de capteurs.

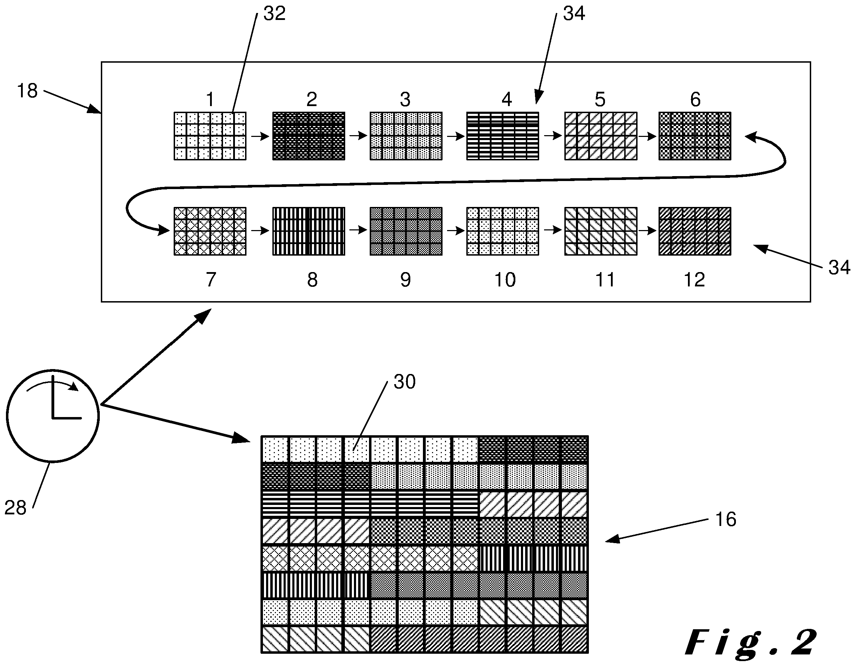

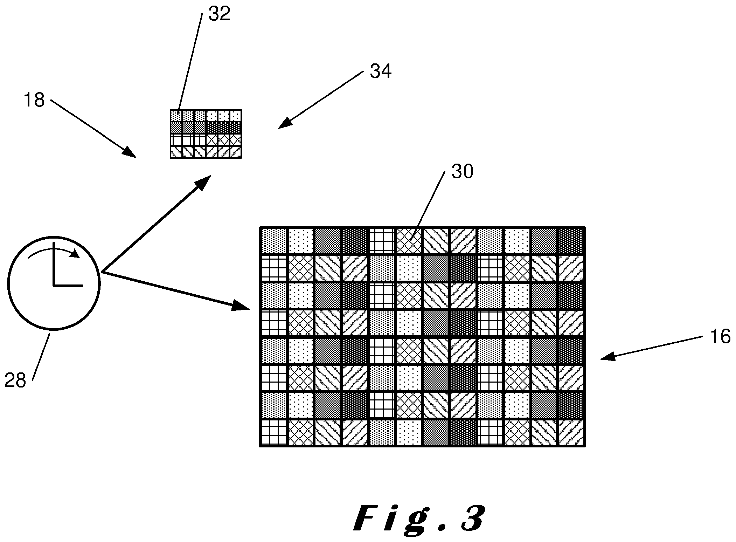

Les figures 2 et 3 montrent un exemple de fonctionnement de l'invention. Sur les figures 2 et 3, un capteur 18 est mis en oeuvre sous forme de caméra 18. L'affichage sur la caméra 18 et l'écran 16 est à balayage, de gauche à droite et de haut en bas.

La figure 2, montre la relation entre les pixels 32 de la caméra 18 et les pixels 30 de l'écran 16 capturant les réactions du sujet. La correspondance graphique représente les pixels qui sont acquis lors de la présentation d'une des images du stimuli. L'unité logique 20 commande les fréquences de rafraîchissement et de capture pour qu'elles soient un multiple l'une de l'autre. Dans cet exemple, l'unité centrale 20 commande la fréquence de la caméra 18 à une valeur plus importante ; sur la durée d'affichage d'une image par l'écran 16, le capteur 18 sous forme de caméra enregistre douze images 34. En d'autres termes, pour huit pixels 30 rafraîchis sur l'écran 16, la caméra 18 capture une image 34. A chaque série de huit pixels 30 rafraîchis sur l'écran 16, la caméra 18 capture une image 34 - soit douze images 34. En outre, l'horloge 28 commande le déclenchement du rafraîchissement des pixels 30 de l'écran 16 et de la capture des images 34 par la caméra 18. La présentation du premier pixel de l'écran 16 commence en même temps que la capture du premier pixel de la première image de la caméra 18. Ainsi, de par les fréquences multiples l'une de l'autre, la première image 34 de la caméra 18 est acquise à l'affichage des huit premiers pixels 30 de l'écran 16 et ainsi de suite. Dans l'exemple de la figure 2, l'écran 16 a une fréquence de rafraîchissement de 1 et la caméra 18 a une fréquence de capture de 12. Il y a donc une relation bijective entre l'instant où un pixel 32 de la caméra 18 est acquis et un pixel 30 de l'écran 16 est affiché, rafraîchi. On obtient donc une synchronisation de la valeur des fréquences et de la phase du déclenchement du rafraîchissement et de la capture.

La figure 3, montre plus en détail la relation entre les pixels 32 de la caméra 18 et les pixels 30 de l'écran 16, avec des fréquences différentes. La correspondance graphique représente les pixels qui sont acquis lors de la présentation d'une des images du stimuli. L'unité logique 20 commande les fréquences de rafraîchissement et de capture pour qu'elles soient un multiple l'une de l'autre. Dans cet exemple, l'unité centrale 20 commande la fréquence de la caméra 18 à une valeur plus importante ; sur la durée d'affichage d'un pixel 30 par l'écran 16, le capteur 18 sous forme de caméra enregistre trois pixels dans l'image 34. En outre, l'horloge 28 commande le déclenchement du rafraîchissement des pixels 30 de l'écran 16 et de la capture de l'image 34 par la caméra 18. La présentation du premier pixel de l'écran 16 commence en même temps que la capture du premier pixel de la première image de la caméra 18. Ainsi, de par les fréquences multiples l'une de l'autre, les trois premiers pixels 32 de la caméra 18 sont acquis à l'affichage du premier pixel 30 de l'écran 16 et ainsi de suite. Dans l'exemple de la figure 3, l'écran 16 a une fréquence de rafraîchissement de 1 et la caméra 18 a une fréquence de capture de 12. Il y a donc une relation bijective entre l'instant où un pixel 32 de la caméra 18 est acquis et un pixel 30 de l'écran 16 est affiché, rafraîchi. On obtient donc une synchronisation de la valeur des fréquences et de la phase du déclenchement du rafraîchissement et de la capture.

Ainsi, le système et procédé de l'invention permettent d'obtenir des informations liées et déterminées par le mode d'affichage par l'écran et de capture par le ou les capteurs. Grâce à l'unité logique et l'horloge centrale - et leur unicité respective - commandant les fréquences et le déclenchement du rafraîchissement et de la capture, il est possible de conclure quel pixel est présenté à un instant et de pouvoir conclure quelle partie de l'image est acquise à cet instant.

## Revendications

1. Système de mesure (12) de réactions d'un sujet (10), comprenant :
- un écran (16) apte à afficher une pluralité de stimuli par un rafraîchissement de pixels de l'écran (16), les stimuli étant aptes à générer des réactions du sujet (10),
- au moins un capteur (18) apte à capturer des images du sujet (10) en réaction aux stimuli,
- une unité logique (20) configurée pour commander le rafraîchissement des pixels de l'écran (16) à une première fréquence et la capture des images du sujet (10) par le capteur (18) à une deuxième fréquence, l'une des fréquences étant un multiple entier de l'autre fréquence, l'unité logique (20) comprenant une horloge centrale (28), le système étant **caractérisé en ce que**
l'horloge centrale (28) est configurée pour commander le rafraîchissement de pixels de l'écran (16) et la capture des images,
dans lequel
- le commandement des fréquences du rafraîchissement des pixels de l'écran (16) et de la capture des images par le capteur (18) est effectué uniquement par l'unité logique (20),
- le commandement du rafraîchissement des pixels de l'écran (16) et de la capture des images par le ou les capteurs (18) est effectuée uniquement par l'horloge centrale (28) de l'unité logique (20),
de sorte qu'il y a une relation bijective entre l'instant où un pixel (32) du capteur (18) est acquis et un pixel (30) de l'écran (16) est rafraîchi.

2. Système de mesure (12) selon la revendication 1, dans lequel le multiple entier est de 1 et l'unité logique (20) est configurée pour commander le rafraîchissement des pixels de l'écran (16) et la capture des images du sujet (10) par le capteur (18) à la même fréquence.

3. Système de mesure (12) selon la revendication 1, dans lequel la première fréquence est plus rapide que la deuxième fréquence, la première fréquence étant un multiple entier de la deuxième fréquence.

4. Système de mesure (12) selon la revendication 1, dans lequel la deuxième fréquence est plus rapide que la première fréquence, la deuxième fréquence étant un multiple entier de la première fréquence.

5. Système de mesure (12) selon l'une quelconque des revendications précédentes, dans lequel l'unité logique (20) est raccordée directement à l'écran (16) et au capteur (18).

6. Système de mesure (12) selon l'une quelconque des revendications précédentes, comprenant deux capteurs (18) sous forme de caméras, chaque capteur (18) capturant des images d'un oeil respectif du sujet (10).

7. Système de mesure (12) selon l'une quelconque des revendications précédentes, dans lequel le au moins un capteur (18) et l'écran (16) sont à balayage de gauche à droite et de haut en bas.

8. Procédé de mesure de réactions d'un sujet (10), comprenant les étapes suivantes :
- la fourniture d'un écran (16), d'au moins un capteur (18), et d'une unité logique (20) comprenant une horloge centrale (28),
- l'affichage par l'écran (16) d'une pluralité de stimuli par un rafraîchissement de pixels de l'écran (16), les stimuli générant des réactions du sujet (10), et la capture par le capteur (18) d'images du sujet (10) en réaction aux stimuli,
le rafraîchissement des pixels de l'écran (16) et la capture des images du sujet par le capteur (18) étant commandés par l'unité logique (20) respectivement à une première fréquence et une deuxième fréquence, l'une des fréquences étant un multiple entier de l'autre fréquence,
**caractérisé en ce que** l'horloge centrale (28) est configurée pour commander le rafraîchissement de pixels de l'écran (16) et la capture des images par le capteur (18),
dans lequel
- le commandement des fréquences du rafraîchissement des pixels de l'écran (16) et de la capture des images par le capteur (18) est effectué uniquement par l'unité logique (20),
- le commandement du rafraîchissement des pixels de l'écran (16) et de la capture des images par le ou les capteurs (18) est effectuée uniquement par l'horloge centrale (28) de l'unité logique (20),
de sorte qu'il y a une relation bijective entre l'instant où un pixel (32) du capteur (18) est acquis et un pixel (30) de l'écran (16) est rafraîchi.

9. Procédé de mesure selon la revendication 8, dans lequel le multiple entier est de 1, l'unité logique (20) commandant le rafraîchissement des pixels de l'écran (16) et la capture des images du sujet (10) par le capteur (18) à la même fréquence.

10. Procédé de mesure selon la revendication 8, dans lequel la première fréquence est plus rapide que la deuxième fréquence, la première fréquence étant un multiple entier de la deuxième fréquence.

11. Procédé de mesure selon la revendication 8, dans lequel la deuxième fréquence est plus rapide que la première fréquence, la deuxième fréquence étant un multiple entier de la première fréquence.

12. Procédé de mesure selon l'une quelconque des revendications 8 à 11, dans lequel :
- l'étape de fourniture comprend la fourniture de deux capteurs (18) sous forme de caméras ;
- l'étape de capture comprend la capture par chaque capteur (18) d'images d'un oeil respectif du sujet (10).

## Patentansprüche

1. System zum Messen (12) von Reaktionen einer Person (10), umfassend:
- einen Bildschirm (16), der imstande ist, eine Vielzahl von Stimuli durch eine Auffrischung von Pixeln des Bildschirms (16) anzuzeigen, wobei die Stimuli imstande sind, Reaktionen der Person (10) zu erzeugen,
- mindestens einen Sensor (18), der imstande ist, Bilder der Person (10) als Reaktion auf die Stimuli aufzunehmen,
- eine Logikeinheit (20), konfiguriert, um die Auffrischung der Pixel des Bildschirms (16) mit einer ersten Frequenz und die Aufnahme der Bilder der Person (10) durch den Sensor (18) mit einer zweiten Frequenz zu steuern, wobei die eine der Frequenzen ein ganzes Vielfaches der anderen Frequenz ist, wobei die Logikeinheit (20) eine Hauptuhr (28) umfasst, wobei das System **dadurch gekennzeichnet ist,**
**dass** die Hauptuhr (28) konfiguriert ist, um die Auffrischung von Pixeln des Bildschirms (16) und die Aufnahme der Bilder zu steuern,
wobei
- die Steuerung der Frequenzen der Auffrischung der Pixel des Bildschirms (16) und der Aufnahme der Bilder durch den Sensor (18) nur durch die Logikeinheit (20) durchgeführt wird,
- die Steuerung der Auffrischung der Pixel des Bildschirms (16) und der Aufnahme der Bilder durch den oder die Sensor(en) (18) nur durch die Hauptuhr (28) der Logikeinheit (20) durchgeführt wird,
sodass eine bijektive Beziehung zwischen dem Zeitpunkt, zu dem ein Pixel (32) des Sensors (18) erfasst wird, und ein Pixel (30) des Bildschirms (16) aufgefrischt wird, besteht.

2. System zum Messen (12) nach Anspruch 1, wobei das ganze Vielfache 1 ist und die Logikeinheit (20) konfiguriert ist, um die Auffrischung der Pixel des Bildschirms (16) und die Aufnahme der Bilder der Person (10) durch den Sensor (18) mit derselben Frequenz zu steuern.

3. System zum Messen (12) nach Anspruch 1, wobei die erste Frequenz schneller ist als die zweite Frequenz, wobei die erste Frequenz ein ganzes Vielfaches der zweiten Frequenz ist.

4. System zum Messen (12) nach Anspruch 1, wobei die zweite Frequenz schneller ist als die erste Frequenz, wobei die zweite Frequenz ein ganzes Vielfaches der ersten Frequenz ist.

5. System zum Messen (12) nach einem der vorstehenden Ansprüche, wobei die Logikeinheit (20) direkt an den Bildschirm (16) und an den Sensor (18) angeschlossen ist.

6. System zum Messen (12) nach einem der vorstehenden Ansprüche, umfassend zwei Sensoren (18) in Form von Kameras, wobei jeder Sensor (18) Bilder eines jeweiligen Auges der Person (10) aufnimmt.

7. System zum Messen (12) nach einem der vorstehenden Ansprüche, wobei der mindestens eine Sensor (18) und der Bildschirm (16) zum Abtasten von links nach rechts und von oben nach unten sind.

8. Verfahren zum Messen von Reaktionen einer Person (10), die folgenden Schritte umfassend:
- die Bereitstellung eines Bildschirms (16), mindestens eines Sensors (18), und einer Logikeinheit (20), die eine Hauptuhr (28) umfasst,
- die Anzeige durch den Bildschirm (16) einer Vielzahl von Stimuli durch eine Auffrischung von Pixeln des Bildschirms (16), wobei die Stimuli Reaktionen der Person (10) erzeugen, und die Aufnahme durch den Sensor (18) von Bildern der Person (10) als Reaktion auf die Stimuli,
wobei die Auffrischung der Pixel des Bildschirms (16) und die Aufnahme der Bilder der Person (18) durch die Logikeinheit (20) jeweils mit einer ersten Frequenz und einer zweiten Frequenz gesteuert werden, wobei die eine der Frequenzen ein ganzes Vielfaches der anderen Frequenz ist,
**dadurch gekennzeichnet, dass** die Hauptuhr (28) konfiguriert ist, um die Auffrischung der Pixel des Bildschirms (16) und die Aufnahme der Bilder durch den Sensor (18) zu steuern,
wobei
- die Steuerung der Frequenzen der Auffrischung der Pixel des Bildschirms (16) und der Aufnahme der Bilder durch den Sensor (18) nur durch die Logikeinheit (20) durchgeführt wird,
- die Steuerung der Auffrischung der Pixel des Bildschirms (16) und der Aufnahme der Bilder durch den oder die Sensor(en) (18) nur durch die Hauptuhr (28) der Logikeinheit (20) durchgeführt wird,
sodass eine bijektive Beziehung zwischen dem Zeitpunkt, zu dem ein Pixel (32) des Sensors (18) erfasst wird, und ein Pixel (30) des Bildschirms (16) aufgefrischt wird, besteht.

9. Verfahren zum Messen nach Anspruch 8, wobei das ganze Vielfache 1 ist, wobei die Logikeinheit (20) die Auffrischung der Pixel des Bildschirms (16) und die Aufnahme der Bilder der Person (10) durch den Sensor (18) mit derselben Frequenz steuert.

10. Verfahren zum Messen nach Anspruch 8, wobei die erste Frequenz schneller ist als die zweite Frequenz, wobei die erste Frequenz ein ganzes Vielfaches der zweiten Frequenz ist.

11. Verfahren zum Messen nach Anspruch 8, wobei die zweite Frequenz schneller ist als die erste Frequenz, wobei die zweite Frequenz ein ganzes Vielfaches der ersten Frequenz ist.

12. Verfahren zum Messen nach einem der Ansprüche 8 bis 11, wobei:
- der Schritt des Bereitstellens das Bereitstellen von zwei Sensoren (18) in Form von Kameras umfasst;
- der Schritt der Aufnahme die Aufnahme durch jeden Sensor (18) von Bildern eines jeweiligen Auges der Person (10) umfasst.

## Claims

1. A system (12) for measuring reactions of a subject (10), comprising:
- a screen (16) capable of displaying a plurality of stimuli via a refresh of pixels of the screen (16), the stimuli being capable of generating reactions from the subject (10),
- at least one sensor (18) capable of capturing images of the subject (10) in reaction to the stimuli,
- a logic unit (20) configured to command the refresh of the pixels of the screen (16) at a first frequency and the capture of the images of the subject (10) by the sensor (18) at a second frequency, one of the frequencies being an integer multiple of the other frequency, the logic unit (20) comprising a central clock (28),
the system being **characterized in that** the central clock (28) is configured to command the refresh of pixels of the screen (16) and the capture of the images,
wherein
- the command of the frequencies for the refresh of the pixels of the screen (16) and for the capture of the images by the sensor (18) is performed solely by the logic unit (20),
- the command of the refresh of the pixels of the screen (16) and of the capture of the images by the sensor or the sensors (18) is performed solely by the central clock (28) of the logic unit (20),
so that there is a bijective relationship between the moment when a pixel (32) on the sensor (18) is acquired and a pixel (30) on the screen (16) is refreshed.

2. The measurement system (12) according to claim 1, wherein the integer multiple is 1 and the logic unit (20) is configured to command the refresh of the pixels of the screen (16) and the capture of the images of the subject (10) by the sensor (18) at the same frequency.

3. The measurement system (12) according to claim 1, wherein the first frequency is faster than the second frequency, the first frequency being an integer multiple of the second frequency.

4. The measurement system (12) according to claim 1, wherein the second frequency is faster than the first frequency, the second frequency being an integer multiple of the first frequency.

5. The measurement system (12) according to any of the preceding claims, wherein the logic unit (20) is connected directly to the screen (16) and to the sensor (18).

6. The measurement system (12) according to any of the preceding claims, comprising two sensors (18) in the form of cameras, each sensor (18) capturing images of a respective eye of the subject (10).

7. The measurement system (12) according to any of the preceding claims, wherein the at least one sensor (18) and the screen (16) are scanned from left to right and top to bottom.

8. A method for measuring reactions of a subject (10), comprising the following steps:
- providing a screen (16), at least one sensor (18), and a logic unit (20) comprising a central clock (28),
- displaying by the screen (16) a plurality of stimuli by a refresh of pixels of the screen (16), the stimuli generating reactions from the subject (10), and capturing by the sensor (18) images of the subject (10) in reaction to the stimuli,
the refresh of the pixels of the screen (16) and the capture of the images of the subject by the sensor (18) being commanded by the logic unit (20) respectively at a first frequency and a second frequency, one of the frequencies being an integer multiple of the other frequency,
**characterized in that** the central clock (28) is configured to command the refresh of pixels of the screen (16) and the capture of the images by the sensor (18),
wherein
- the command of the frequencies for the refresh of the pixels of the screen (16) and for the capture of the images by the sensor (18) is performed solely by the logic unit (20),
- the command of the refresh of the pixels of the screen (16) and of the capture of the images by the sensor or the sensors (18) is performed solely by the central clock (28) of the logic unit (20),
so that there is a bijective relationship between the moment when a pixel (32) on the sensor (18) is acquired and a pixel (30) on the screen (16) is refreshed.

9. The measurement method according to claim 8, wherein the integer multiple is 1, the logic unit (20) commanding the refresh of the pixels of the screen (16) and the capture of the images of the subject (10) by the sensor (18) at the same frequency.

10. The measurement method of claim 8, wherein the first frequency is faster than the second frequency, the first frequency being an integer multiple of the second frequency.

11. The measurement method of claim 8, wherein the second frequency is faster than the first frequency, the second frequency being an integer multiple of the first frequency.

12. The measurement method according to any one of claims 8 to 11, wherein:
- the providing step comprises providing two sensors (18) in the form of cameras;
- the capturing step comprises the capture by each sensor (18) of images of a respective eye of the subject (10).
